(19) Europäisches Patentamt
European Patent Office
Office européen des brevets

(11) Veröffentlichungsnummer: **0 286 703**
**A1**

(12) # EUROPÄISCHE PATENTANMELDUNG

(21) Anmeldenummer: 87105487.0

(22) Anmeldetag: 14.04.87

(51) Int. Cl.⁴: **C07C 65/105 , C07C 65/34 , C07C 69/78 , C07C 103/84 , A61K 31/19 , A61K 31/235**

(43) Veröffentlichungstag der Anmeldung:
**19.10.88 Patentblatt 88/42**

(84) Benannte Vertragsstaaten:
**AT BE CH DE ES FR GB GR IT LI LU NL SE**

(71) Anmelder: **Klinge Pharma GmbH**
**Berg-am-Laim-Strasse 129**
**D-8000 München 80(DE)**

(72) Erfinder: **Grill, Helmut**
**Zugspitzstrasse 146**
**D-8011 Vaterstetten(DE)**
Erfinder: **Reiter, Friedemann**
**Zugspitzstrasse 36**
**D-8011 Putzbrunn(DE)**
Erfinder: **Schliack, Michael**
**Neumarkter Strasse 82**
**D-8000 München 80(DE)**
Erfinder: **Löser, Roland**
**Fichtenweg 2**
**D-8133 Feldafing(DE)**
Erfinder: **Seibel, Klaus**
**Haberlstrasse 9**
**D-8032 Gräfelfing(DE)**

(74) Vertreter: **Freiherr von Pechmann, Eckehart et al**
**Patentanwälte Wuesthoff- v. Pechmann-Behrens-Goetz Schweigerstrasse 2**
**D-8000 München 90(DE)**

(54) **Neue Benzoesäure-Derivate, sowie Verfahren zu ihrer Herstellung und ihre Verwendung als Arzneimittel.**

(57) Parasubstituierte Benzoesäurederivate der allgemeinen Formel (1)

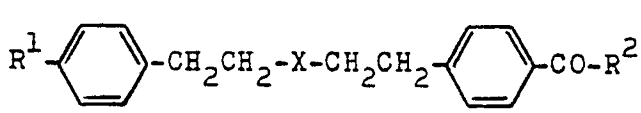

(1)

und ihre physiologisch verträglichen Salzen, worin R' = H,Isopropyl, t-Butyl

X = - CH -, - C -
      OH      O

$R^2$ = -OH, -OR' sein kann, wobei R' ein geradkettiger gegebenenfalls verzweigter gesättigter oder ungesättigter $C_1$ bis $C_3$ Alkylrest oder eine $-NHCH_2COOH$-Gruppe ist.

EP 0 286 703 A1

besitzen hypolipämische Wirkung. Sie können nach an sich bekannten Methoden sowohl über entsprechende ungesättigte Ketoverbindungen als auch gesättigte Hydroxyverbindungen gewonnen werden, wobei der ansich veresterte Benzoesäurerest auch verseift bzw. in ein Carboxymethylamid übergeführt werden kann.

Zur medikamentösen Behandlung von Fettstoffwechselstörungen sind bereits zahlreiche Wirkstoffe in die Therapie eingeführt worden. Einer der ersten Vertreter aus der Gruppe der Aryloxy-isoalkansäuren war das CLOFIBRAT. Auf Grund seiner schwachen Wirksamkeit wurde sowohl eine Reihe von Derivaten wie z.B. ETOFIBRAT oder ETOFYLLINCLOFIBRAT als auch Strukturanaloga wie z.B. BEZAFIBRAT, FENOFIBRAT oder GEMFIBROZIL entwickelt, die jedoch in ihren unerwünschten Wirkungen dem CLOFIBRAT ähneln, was auf einen ähnlichen Wirkungsmechanismus schließen läßt. Bekanntgewordene Nebenwirkungen sind z.B. Magen-Darm-Beschwerden, Appetitlosigkeit, Übelkeit, Allergien, Myositis, Myalgien und Potenzstörungen sowie Erhöhungen des Serumkreatinins, Serumharnstoffs und des lithogenen Index, eine Senkung der alkalischen Phosphatase, ein Anstieg der Kreatinin-Phosphokinase und eine Stimulation der Peroxisomenbildung.

Eine Beseitigung dieses bisherigen Mangels erscheint nur durch Auffinden neuer Wirkstoffe möglich, die auf Grund ihrer deutlich abweichenden Struktur veränderte biologische Eigenschaften erwarten lassen. Überraschenderweise wurde nun gefunden, daß Benzoesäurederivate der im Patentanspruch 1 angegebenen Formel (1), eine gegenüber BEZAFIBRAT deutlich überlegene hypolipämische Wirkung besitzen. Es handelt sich um parasubstituierte Phenylpentanol-bzw. Phenylpentanonbenzoesäureverbindungen, die zu einer bisher nicht beschriebenen Strukturreihe gezählt werden können.

Bekannt sind zwar in Paraposition veretherte Benzoesäuren, die auch hypolipämische Eigenschaften besitzen. Dazu zählen Verbindungen, die sich von Ethern des Glycerins (DE-PS 24 60 689), des 1,3-Dihydroxyacetons (DE-OS 27 35 856) und anderen Alkoholen (DE-OS 33 25 164; US-PS 4,067,892; US-PS 4,154,850) ableiten. Auf Grund ihrer Etherfunktion muß jedoch mit veränderten biologischen Eigenschaften gerechnet werden.

Die Herstellung dieser neuen, hypolipämisch wirkenden Verbindungen kann man in an sich bekannter Weise durchführen, wobei man erfindungsgemäß die in Anspruch 2 angegebenen Verfahren anwendet. Die zur Synthese der erfindungsgemäßen Verbindungen notwendigen Ausgangsprodukte lassen sich beispielsweise nach folgenden Verfahren herstellen.

Durch Kondensation von Aceton mit Aldehyden der allgemeinen Formel (7)

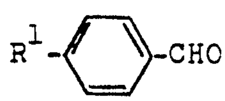 (7)

in der $R^1$ die in Anspruch 1 angegebene Bedeutung besitzt, werden Benzalacetone der allgemeinen Formel (8) erhalten.

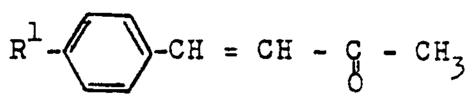 (8)

Eine Umsetzung mit 4-Formylbenzoesäurealkylester der allgemeinen Formel (9)

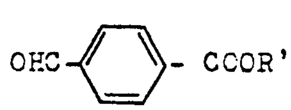 (9)

in der R' die in Anspruch 1 angegebene Bedeutung besitzt, führt zu 1,4-Pentadienonen der allgemeinen Formel (2)

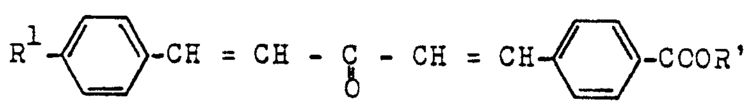 (2)

die sich in Gegenwart von Tris(triphenylphosphin)ruthenium-(II)-chlorid mit Benzylalkohol selektiv zu den erfindungsgemä&en Verbindungen der Formel (3)

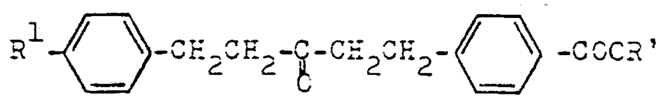

(3)

hydrieren lassen.

Durch alkalische Verseifung und anschließendem Ansäuern mit Mineralsäure werden daraus die erfindungsgemäßen Verbindungen der allgemeinen Formel (10) erhalten.

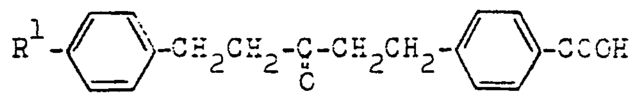

(10)

Verbindungen der allgemeinen Formel (3) lassen sich in der Kälte mit Natriumboranat zu den erfindungsgemäßen Verbindungender allgemeien Formel (4)

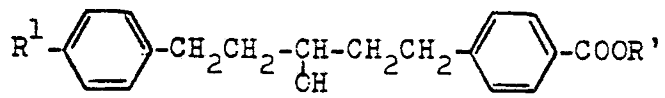

(4)

reduzieren, und durch alkalische Verseifung und anschließendem Ansäuern mit Mineralsäure in die erfindungsgemäßen Säuren der allgemeinen Formel (11) überführen.

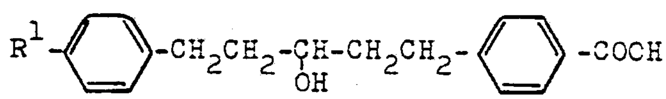

(11)

Eine Umsetzung mit Acetylchlorid liefert Verbindungen der allgemeinen Formel (12)

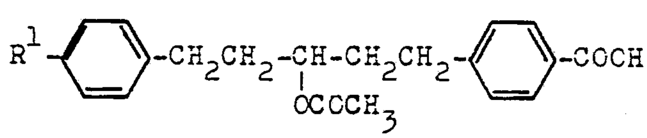

(12)

die sich mit Thionylchlorid in Säurechloride der allgemeinen Formel (13)

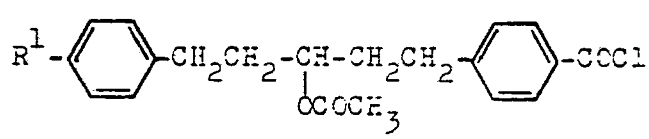

(13)

überführen lassen.

Aus den Säurechloriden der allgemeinen Formel (13) werden nach Umsetzung mit Natriumglycinat und anschließender alkalischer Verseifung, durch Ansäuern mit Mineralsäure die erfindungsgemäßen Verbindungen der allgemeinen Formel (14) erhalten

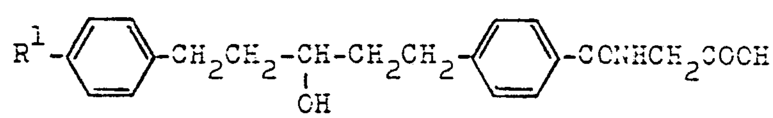

(14)

Durch selektive Oxidation mit Dimethylsulfoxid werden Verbindungen der allgemeinen Formel (4), (11) und (14) in die erfindungsgemäßen Verbindungen der allgemeinen Formel (15) übergeführt.

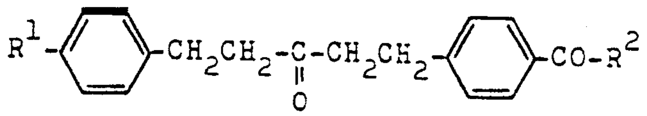 (15)

in der $R^1$ und $R^2$ die in Anspruch 1 angegebene Bedeutung besitzen.

Die Überlegenheit der beanspruchten Verbindungen gegenüber dem bereits seit langem in die Therapie eingeführten BEZAFIBRAT läßt sich anhand der lipidsenkenden Wirkung eindeutig belegen.

Die lipidsenkende Wirkung wurde an Gruppen von jeweils 10 normolipämischen, männlichen, 200 bis 220 g schweren Wistar-Ratten geprüft.

Die Durchführung der Teste erfolgte nach einem 3-wöchigen Umtrainieren der Freßgewohnheiten der Tiere.Die kontrollierte Fütterung wurde täglich vcn 10.00 bis 12.00 Uhr durchgeführt. Die Applikation der Testsubstanzen erfolgte um 11.00 Uhr.

Die Testverbindungen wurden in einer wäßrigen Lösung von 0.25% Agar und 0.84% Natriumchlorid aufgenommen und oral verabreicht. Nach Applikation von 3 x 100 mg/kg über einen Zeitraum von drei Tagen wurde den Tieren Blut entnommen.

Die Bestimmung von Cholesterin und Triglyceriuen wurde mit Hilfe des Zentrifugalanalysers "Cobas Bio " von Hoffman-La Roche vorgenommen.

Methoden :

a) Cholesterinbestimmung CHOD-PAP-Methode; enzymatischer Farbtest nach J. Siedel et al [J.Clin.einem.Clin.Biochem. 19,838 (1981)]

b) Triglyceridbestimmung Enzymatische Spaltung der Triglyceridc mit Hilfe spezieller Lipasen mit nachfolgender enzymatischer Bestimmung des freigewordenen Glycerins.

[H.U. Bergmeyer,"Methoden der enzymatischen Analyse",3.Auflage, Bd.II,Verlag Chemie,Weinheim, 1974,Seite 1878]

Tabelle 1

Prozentuale Änderung der Gesamtcholesterin (TC)- und Triglycerid (TG)-Spiegel im Rattenserum nach oraler Applikation der Testsubstanzen

| Verbindungsnummer | % Änderung | | | | | |
|---|---|---|---|---|---|---|
| | TC | | | TG | | |
| | $\bar{X}$ | $\pm$ | $S_x$ | $\bar{X}$ | $\pm$ | $S_x$ |
| Vergleichssubstanz BEZAFIBRAT | - 22.3 | ± | 15.8 | + 6.1 | ± | 9.9 |
| 2 | - 29.0 | ± | 10.8 | - 42.6 | ± | 12.8 |
| 3 | - 26.1 | ± | 17.2 | - 21.9 | ± | 11.5 |
| 4 | - 26.3 | ± | 13.9 | - 42.0 | ± | 7.0 |
| 5 | - 17.6 | ± | 15.0 | - 37.4 | ± | 12.7 |
| 6 | - 9.1 | ± | 5.6 | - 25.3 | ± | 20.4 |
| 7 | - 35.1 | ± | 9.7 | - 31.3 | ± | 13.0 |
| 8 | - 23.1 | ± | 13.1 | - 38.5 | ± | 13.0 |
| 9 | - 35.6 | ± | 20.9 | - 64.6 | ± | 15.8 |
| 10 | - 15.4 | ± | 10.7 | - 31.3 | ± | 25.6 |
| 11 | - 26.1 | ± | 7.2 | - 35.4 | ± | 14.7 |
| 13 | - 6.9 | ± | 21.7 | - 15.7 | ± | 14.8 |
| 14 | - 24.8 | ± | 18.4 | - 45.4 | ± | 12.4 |
| 15 | - 28.0 | ± | 7.7 | - 20.1 | ± | 7.1 |

Für die therapeutische Anwendung als hypolipämische Arzneimittel werden die neuen Verbindungen der allgemeinen Formel (1) und ihre Salze bevorzugt oral verabreicht.Gewöhnlich beträgt die orale Tagesdosis bei Erwachsenen 0.1 bis 1 g.

Die Wirkstoffe können zur oralen Verabreichung in üblicher Weise galenisch verarbeitet werden. Als pharmazeutische Trägerstoffe eignen sich gängige Hilfsstoffe wie Lactose,Saccharose, Mannit, Kartoffel- oder Maisstärke, Cellulosederivate oder Gelatine, gegebenenfalls unter Zusatz von Gleitmitteln, wie z.B. Magnesium-oder Calciumstearat, sowie Polyethylenglykole.

Bevorzugte Verabreichungsformen sind Steckkapseln aus Hartgelatinesowie geschlossene Weichgelatinekapseln. Die Freisetzung der beanspruchten Verbindungen kann je nach pharmazeutischer Verarbeitung beschleunigt oder verzögert werden.

In Steckkapseln kann gegebenenfalls der reine Wirkstoff evtl. mit einem geringen Zusatz an Gleitmitteln enthalten sein .Bei Konfektionierung in Weichgelatinekapseln kann der reine Wirkstoff in geeigneten Flüssigkei&en gelöst oder suspendiert werden, so z.B. in flüssigen Pc&yethylenglykolen oder Pflanzenölen. Bei entsprechenden physikalischen Eigenschaften des Wirkstoffes wird eine Verarbeitung zu Granulaten

bevorzugt.

Die Herstellung der Synthese-Vorstufen erfolgt nach bekannten Verfahren wie sie nachfolgend anhand von einigen Beispielen beschrieben werden.

Beispiel 1

4-[5-(4'-Isopropylphenyl)-3-oxopentyl]benzoesäuremethylester

a) 4-[5-(4'-Isopropylphenyl)-3-oxo-1.4-pentadienyl]benzoesäuremethylester

94.1 g (0.50 Mol) 4'-Isopropylbenzalaceton und 91.8 g (0.56 Mol) 4-Formylbenzoesäuremethylester werden unter Stickstoff in 500 mL Methanol gelöst und unter Rühren innerhalb 5 Minuten mit 45 mL 2 Natronlauge versetzt. Nach einer Stunde fügt man nochmals 45 mL 2 N Natronlauge hinzu und rührt weitere drei Stunden. Das abgeschiedene Prod&:t wird abgesaugt, mit kaltem Methanol neutral gewaschen und aus 675 mL Methanol unter Zusatz von 70 mL Ethylacetat umkristallisiert. Hellgelbe Kristalle vom Schmp. 113 - 115°C[1)]Ausbeute 84.9 g (51%)

$^1$H-NMR-Spektrum ($CDC_3$) [2)]:

1.27 d (6) $(CH_3)_2CH$

2.93 m (1) $(CH_3)_2CH$

3.93 s (3) $CH_3O$

6.80 bis 8.20 m (12) Aromat,4 =CH-

[1)]Die Schmelzpunkze wurden mit einem Kofler-Heiztisch-Mikroskop ermittelt und sind nicht korrigiert.

[2)]Aufgenommen bei 60 MHz

Die chemischen Verschiebungen sind in ppm gegen TMS ($\delta$=0.o) angegeben, die relativen Intensitäten sind in Klammern beigefügt. s = Singulett, d = Dublett, t = Triplett, q = Quartett, m = Multiplett.

b) Erfindungsgemäße Herstellung von 4-[5-(4'-Isopropylphenyl)-3-oxopentyl]benzoesäuremethylester

33.4 g(0.10 Mol) 4-[5-(4'-Isopropylphenyl)-3-oxo-1,4-pentadienyl]benzoesäuremethylester werden in 40 mL (0.385 Mol) Benzylalkohol gelöst, unter Stickstoff mit 0.20 g (0.2 mMol) Tris(triphenylphosphin)-ruthenium(II)-chlorid versetzt und am Luftkühler im schwachen Stickstoffstrom zwei Stunden auf 190 - 200°C (Bad-Temperatur) erhitzt.Anschließend wird der entstandene Benzaldehyd samt noch vorhandenem Benzylalkohol im Vakuum entfernt und der Rückstand zur Vervollständigung der Reduktion nochmals mit 30 mL frischem Benzylalkohol und 0.15 g Katalysator weitere zwei Stunden unter Stickstoff auf 190 -200°C (Bad-Temperatur) erhitzt. Die flüchtigen Anteile werden dann bei 0.1 mbar und 1.00°C (Bad-Temperatur) entfernt und der rotbraune ölige Rückstand in Dichlormethan aufgenommen.Nach säulenchromatographischer Reinigung an Kieselgel mit Dichlormethan, wird das Lösungsmittel im Vakuum abgezogen und der Rückstand bei 0.03 mbar und 225°C im Kugelrohrofen destilliert.Farbloses Öl mit einem Brechungsindex von $n_D^{25}$ = 1.5445.Ausbeute 11.2 g (33 %).

$C_{22}H_{26}O_3$ (338.4)

Mol.-Gew : 338 (massenspektrometrisch bestimmt)[X]

IR-Spektrum (Film) : $\nu$(C=O) 1720, 1710 $cm^{-1}$

$^1$H-NMR-Spektrum ($CDCl_3$):

1.20 s (6) $(CH_3)_2CH$

2.47 bis 3.13 m (9) 2 $ArCH_2CH_2$ und $(CH_3)_2CH$

3.90 s (3) $OCH_3$

6.93 bis 8.10 m (8) Aromat

[X)] mittels Elektronenstoß-Ionisation (70 eV)

Beispiel 2

4 = [5-(4'-Isopropylphenyl)-3-hydroxypentyl]benzoesäure

34.0 g (0.10 Mol) roher 4-[5-(4'-Isopropylphenyl)-3-oxopentyl]-benzoesäuremethylester, dargestellt nach Beispiel 1, werden in 100 ml Ethanol gelöstund bei 0 - 10° C langsam mit einer eiskalten Lösung von 2.0 g (0.053 Mol) Natriumboranat in 10 mL Wasser versetzt.Nach erfolgter Zugabe entfernt man die Kühlung und rührt noch 2.5 Stunden bei Raumtemperatur.Anschließend fügt man 13 g Kaliumhydroxid hinzu, rührt weitere drei Stunden bei Raumtemperatur und läßt über Nacht stehen. Nach Verdünnung des Reaktionsgemisches mit Wasser wird zweimal mit Ether extrahiert, die wäßrige Phase mit konzentrierter Salzsäure angesäuert und das abgeschiedene Produktin Ethylacetat aufgenommen. Man wäscht mit Wasser neutral, trocknet über Natriumsulfat und entfernt das Lösungsmittel im Vakuum. Der Rückstand wird über Kieselgel mit $CH_2Cl_2$ $CH_3OH$ (95/5) chromatographisch gereinigt und nach Abziehen des Lösungsmittels aus Acetonitril kristallisiert. Farblose Kristalle vom Schmp. 104 - 106° C. Ausbeute 12.7 g (39 %).

$C_{21}H_{26}O_3$ (326.4)

Mol.-Gew. : 326 (massenspektrometrisch bestimmt)

IR-Spektrum (KBr) :
$\nu$(OH) 3500 bis 2400 $cm^{-1}$
$\nu$(C-C) 1685 $cm^{-1}$
$^1$H-NMR-Spektrum ($CDCl_3$) :
1.23 d (6) $(CH_3)_2CH$
1.57 bis 2.07 m (4) 2 $ArCH_2CH_2$
2.50 bis 3.03 m (5) 2 $ArCH_2CH_2$ und $(CH_3)_2CH$
3.67 m (1) CH(OH)
6.67 breites s (2) OH, COOH
7.03 bis 8.13 m (8) Aromat

Beispiel 3

4-[5-(4'-tert.Butylphenyl)-3-hydroxypentyl]benzoesäureisopropylester

34.0 g (0.10 Mol) 4-[5-(4'-tert.Butylphenyl)-3-hydroxypentyl]benzoesäure, dargestellt analog den Beispielen 1 und 2, werden in 300 mL Isopropanol mit 5 mL konzentrierter Schwefelsäure 20 Stunden unter Rückfluß gekocht.Nach dem Abkühlen wird das Reaktionsgemisch in Eiswasssr gegossen und zweimal mit Dichlormethan ausgeschüttelt. Die organische Phase wird zunächst mit Wasser, dann mit wässriger Natriumhydrogenkarbonatlösung und schließlich nochmals mit Wasser neutral gewaschen. Nach dem Trocknen über Natriumsulfat, entfernt man das Lösungsmittel im Vakuum und reinigt den farblosen Rückstand chromatographisch mit $CH_2Cl_2/CH_3OH$ (98/2) an Kieselgel.Nach Abziehen des Lösungsmittels im Vakuum, wird aus Petrolether (40-60°C Fraktion) kristallisisrt.Farblose Kristalle vom Schmp. 47-48°C Ausbeute 18.4 g (48%)

$C_{25}H_{34}O_3$ (382.5)

Mol.-Gew. : 382 (massenspektrometrisch bestimmt)

IR-Spektrum (KBr):
$\nu$(OH) 3600 bis 2800 $cm^{-1}$
$\nu$(C-O) 1720 $cm^{-1}$
$^1$H-NMR-Spektrum ($CDCl_3$):
1.30 s (15) $(CH_3)_3C$
1.33 d (15) $(CH_3)_2CHO$
1.53 bis 2.07 m (5) $2ArCH_2CH_2$ und OH(austauschbar mit $D_2O$)
2.50 bis 3.00 m (4) $2ArCH_2CH_2$
3.60 m (1) CH(OH)
5.23 m (1) $(CH_3)_2CHO$
6.53 bis 8.10 (8) Aromat

Beispiel 4

N-Carboxymethyl-f4-[5-(4′-tert.butylphenyl)-3-hydroxypentyl]benzamid

a) 4-[5-(4′-tert.Butylphenyl)-3-acetoxypentyl]benzoesäure

34.0 g (0.10 Mol) 4-[5-(4' tert.Butylphenyl)-3-hydroxypentyl]benzoesäure und 0.30 g (2.2 mMol) wasserfreies Zinkchlorid werden in 50 mL wasserfreier Essigsäure suspendiert und mit 14.3. mL (0.20 Mol) Acetylchlorid versetzt, wobei eine klare Lösung entsteht.Nach zweistündigem Rühren bei Raumtemperatur gießt man die Reaktionslösung in Eiswasser. Das abgeschiedene Produkt wird in Chloroform aufgenommen und mit Wasser gewaschen. Nach dem Trocknen über Natriumsulfat wird das Lösungsmittel im Vakuum entfernt und das farblose,kristallisierende Rohprodukt ohne Reinigung zur weiteren Umsetzung verwendet. Rohausbeute 38.3 g (100 %)

$^1$H-NMR-Spektrum ($CDCl_3$):
1.30 s (9) $(CH_3)_3C$
1.63 bis 2.13 m (7) $2ArCH_2CH_2$ und $CH_3CO$
2.0 s (7) $2ArCH_2CH_2$ und $CH_3CO$
2.37 bis 2.90 m (4) $2\ ArCH_2CH_2$
4.97 m (1) $CH(OCOCH_3)$
6.83 bis 8.10 m (8) Aromat
11.83 breites s (1) COOH

b) Erfindungsgemäße Herstellung von N-Carboxymethyl-4-[5-(4′-tert.butylphenyl)-3-hydroxypentyl]benzamid

38.3 g (0.10 Mol) rohe 4-[5-(4′-tert.Butylphenyi)-3-acetoxypentyl]benzoesäure und 12 mL (0.165 Mol) Thionylchlorid in 200 mL Toluol werden 3.5 Stunden unter Rückfluß erhitzt. Anschließend destilliert man Lösungsmittel und überschüssiges Thionylchlorid im Vakuum ab und nimmt den öligen Rückstand in 250 ml trockenem Dioxan auf. Diese Lösung tropft man unter lebhaftem Rühren bei 5 bis 7° C in eine Lösung von 29.5g (0.39 Mol) Glycin und 15.6 g (0.39 Mol) Natriumhydroxid in 350 mL Wasser, rührt dann drei Stunden bei Raumtemperatur und läßt über Nacht stehen.Dann fügt man 20 g Natriumhydroxid hinzu und rührt weitere 3.5 Stunden bei Raumtemperatur.Die Reaktionslösung wird schließlich mit Wasser verdünnt und nach dem Ansäuern mit konzentrierter Salzsäure das ausgefällte Endprodukt in Ethylacetat aufgenommen. Nach zweimaligem Waschen der organischen Phase wird über Natriumsulfat getrocknet,das Lösungsmittel im Vakuum entfernt und der Rückstand aus Acetonitril kristallisiert. Farblose Kristalle vom Schmp. 133 -135° C. Ausbeute 32.2 g (81 %)

$C_{24}H_{31}NO_4$ (397.5)

Mol.-Gew. 397 (massenspektrometrisch bestimmt)

IR-Spektrum (KBr)
$\nu$(OH) 3600 bis 2400 $cm^{-1}$
$\nu$(C-O) 1725 $cm^{-1}$
$\nu$(NH) 3360 $cm^{-1}$

$^1$H-NMR-Spektrum ($d_6$-Aceton):
1.23 s (9) $(CH_3)_3C$
1.57 bis 2.03 m (4) $2\ ArCH_2CH_2$
2.50 bis 3.03 m (4) $2\ ArCH_2CH_2$
3.60 m (1) CH(OH)
4.10 4.20 2 s (2) $NCH_2$
6.47 bis 8.17 m (11) Aromat,NH,OH, COOH

In analoger Weise zu den Beispielen wurden weitere Verbindungen der allgemeinen Formel (1) hergestellt und in nachfolgender Tabelle mit den Schmelzpunkten aufgeführt. Aus Gründen der Vollständigkeit werden in folgender Tabelle die in den Beispielen beschriebenen Verbindungen nochmals angegeben.

Tabelle 2

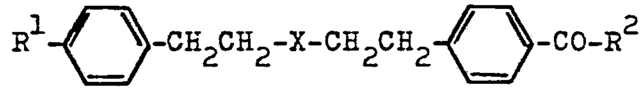

| Nr. | $R^1$ | X | $R^2$ | Schmp. (°C)[x)] | Lösungsmittel[xx)] |
|---|---|---|---|---|---|
| 1 | H | CO | OH | 140-141 | (a) |
| 2 | $(CH_3)_2CH$ | CO | $OCH_3$ | $n_D^{25}$=1.5445 (Öl)[xxx)] | |
| 3 | $(CH_3)_3C$ | CO | OH | 139-140 | (f) |
| 4 | $(CH_3)_3C$ | CO | $OCH_3$ | 47- 48 | (d) |
| 5 | H | CHOH | OH | 99-100 | (a) |
| 6 | H | CHOH | $OCH_2CH_2CH_3$ | 48- 49 | (b) |
| 7 | $(CH_3)_2CH$ | CHOH | OH | 104-106 | (a) |
| 8 | $(CH_3)_2CH$ | CHOH | $OCH_2CH{=}CH_2$ | 55- 56 | (d) |
| 9 | $(CH_3)_3C$ | CHOH | OH | 122-123 | (a) |
| 10 | $(CH_3)_3C$ | CHOH | $OCH_2CH_3$ | 46- 47 | (d) |
| 11 | $(CH_3)_3C$ | CHOH | $OCH(CH_3)_2$ | 47- 48 | (d) |
| 12 | H | CO | $NHCH_2COOH$ | 126-128 | (a) |
| 13 | H | CHOH | $NHCH_2COOH$ | 97- 99 | (c) |
| 14 | $(CH_3)_2CH$ | CHOH | $NHCH_2COOH$ | 116-119 | (e) |
| 15 | $(CH_3)_3C$ | CHOH | $NHCH_2COOH$ | 133-135 | (a) |

x) Die Schmelzpunkte wurden mit einem Kofler-Heiztisch-Mikroskop ermittelt und sind nicht korrigiert

xx) Kristalle aus (a) : Acetonitril
(b) : 1-Chlorbutan/Petrolether (40-60)
(c) : Ethylacetat/Diisopropylether
(d) : Petrolether (40-60)
(e) : Acetonitril/Chloroform
(f) : 1-Chlorbutan

xxx) Siedepunkt 225°C/0.03 mbar (Kugelrohr)

Beispiel 5

4-[5-(4'-Isopropylphenyl)-3-oxopentyl]benzoesäuremethylester enthaltendes Arzneimittel

400 g 4-[5-(4'-Isopropylphenyl)-3-oxopentyl]benzoesäuremethylester werden mit 200 g Polyethylenglykol vsrmischt und nach dem Schererverfahren in eintausend Weichgelatinekapseln abgefüllt, die je 400 mg Wirkstoff enthalten .

Beispiel 6

4-[5-(4'-tert.Butylphenyl)-3-hydroxypentyl]benzoesäure enthaltendes Arzneimittel

500 g 4-[5-(4'-tert.Butylphenyl)-3-hydroxypentyl]benzoesäure werden nach feinem Puvsrisieren mit Talkum und Calciumstearat versetzt und nach sorgfältiger Durchmischung in zweitausend Hartgelatinekapseln abgefüllt, sodaß jede Kapsel 250 mg Wirkstoff enthält.

## Ansprüche

1. Benzoesäurederivate der allgemeinen Formel (1)

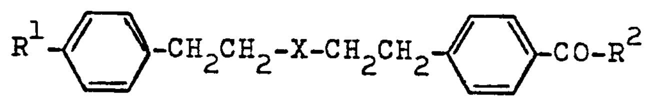

(1)

und ihre physiologisch verträglichen Salze, worin
$R^1$ = H, Isopropyl, t-Butyl
X - CH(OH) -, - C(=O) - und
$R^2$ = -OH, -OR' sein kann, wobei R' ein geradkettiger verzweigter gesättigter oder ungesättigter $C_1$ bis $C_3$-Alkylrest oder eine $-NHCH_2COOH$-Gruppe ist.

2. Verfahren zur Herstellung von Benzoesäurederivaten nach Anspruch 1, dadurch gekennzeichnet, daß man

a) Verbindungen der allgemeinen Formel (2)

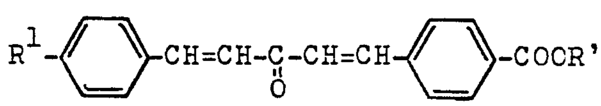

(2)

in der $R^1$ und R' die in Anspruch 1 beschriebene Bedeutung besitzen mit Benzylalkohol in Gegenwart von Tris(triphenylphosphin)ruthenium-(II)-chlorid zu den beanspruchten Verbindungen der allgemeinen Formel (3)

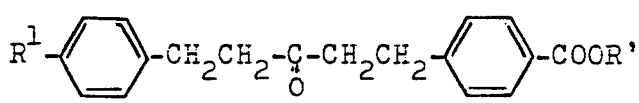

(3)

in der $R^1$ und R' die in Anspruch 1 beschriebene Bedeutung besitzen, umsetzt, gegebenenfalls mit Alkali verseift, durch Zugabe von Mineralsäure die Carboxylverbindung in Freiheit setzt und nach Kristallisation wenn erforderlich durch Zugabe von Basen die entsprechenden Salze herstellt.

b) Verbindungen der allgemeinen Formel (3)

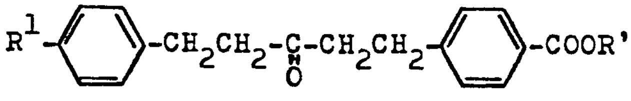

(3)

in der $R^1$ und R' die in Anspruch 1 beschriebene Bedeutung besitzen , mit Natriumboranat zu den entsprechenden Alkoholen der allgemeinen Formel (4)

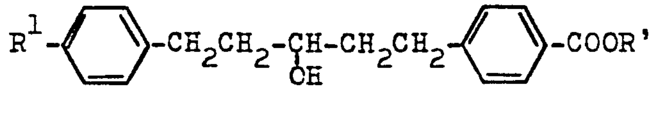

(4)

umsetzt, wobei $R^1$ und R′ die oben angegebene Bedeutung besitzen, gegebenenfalls mit Alkali verseift, durch Zugabe von Mineralsäure die Carboxylverbindung in Freiheit setzt und nach Kristallisation, wenn erforderlich, durch Zugabe von Basen die entsprechenden Salze gewinnt:

c)Verbindungen der allgemeinen Formel (5)

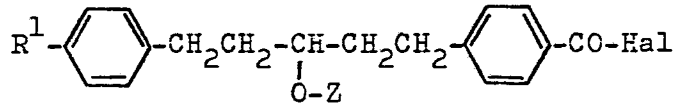
(5)

in der $R^1$ die in Anspruch 1 beschriebene Bedeutung besitzt, Z eine Schutzgruppe, z.B. Acetyl darstellt und Hal ein Halogenatom, bevorzugt Chlor bedeutet, mit Natriumglycinat in an sich bekannter Weise umsetzt, anschließend mit Alkali die Schutzgruppe abspaltet, durch Zugabe von Mineralsäure die Carboxylverbindung in Freiheit setzt und nach Kristallisation gegebenenfalls mit Basen zu den entsprechenden Salzen umsetzt;

d)Verbindungen der allgemeinen Formel (6)

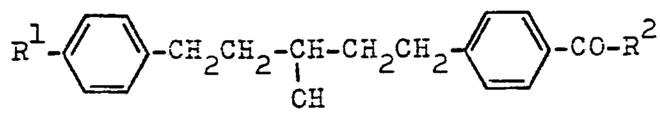
(6)

in der $R^1$ und $R^2$ die in Anspruch 1 beschriebene Bedeutung besitzen einer Oxidation mit Dimethylsulfoxid unterzieht, das Oxidationsprodukt kristallisiert und gegebenenfalls die entsprechenden Carboxylverbindungen mit Basen zu den Salzen umsetzt.

3.Arzneimittel,gekennzeichnet durch den Gehalt einer Verbindung nach Anspruch 1, neben pharmazeutisch anwendbaren Verdünnungs-oder Trägermitteln.

Patentansprüche für die folgenden Vertragsstaaten: AT und ES

1. Verfahren zur Herstellung von Benzoesäurederivaten der allgemeinen Formel (1)

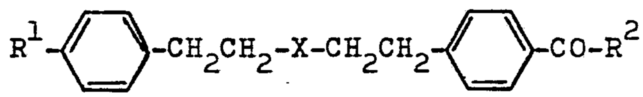
(1)

und ihre physiologisch verträglichen Salze, worin

$R^1$ = H, Isopropyl, t-Butyl

X = $-\underset{OH}{CH}-$, $-\underset{O}{\overset{}{C}}-$ und

$R^2$ = -OH, -OR′ sein kann, wobei R′ ein geradkettiger verzweigter gesättigter oder ungesättigter C· bis $C_3$-Alkylrest oder eine $-NHCH_2COOH$-Gruppe ist,

dadurch **gekennzeichnet,** daß man

a) eine Verbindung der allgemeinen Formel (2)

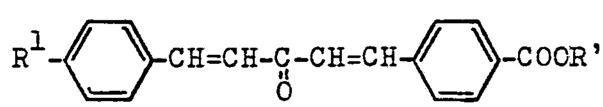
(2)

in der R′ und R′ die oben angegebene Bedeutung besitzen, mit Benzylalkohol in Gegenwart von Tris-(triphenylphosphin)ruthenium-(II)-chlorid zu der Verbindung der allgemeinen Formel (3)

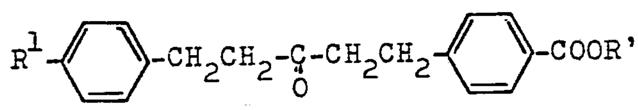
(3)

in der R' und R' die oben angegebene Bedeutung besitzen, umsetzt, gegebenenfalls mit Alkali verseift, durch Zugabe von Mineralsäure die Carboxylverbindung in Freiheit setzt und nach Kristallisation, wenn erforderlich, durch Zugabe von Base die entsprechenden Salze herstellt;

b) eine Verbindung der allgemeinen Formel (3)

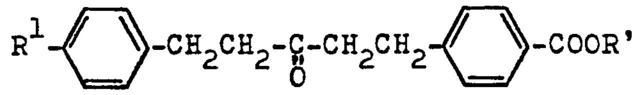

(3)

in der R' und R' die oben angegebene Bedeutung besitzen, mit Natriumboranat zu den entsprechenden Alkoholen der allgemeinen Formel (4)

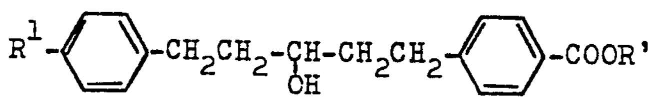

(4)

umsetzt, wobei R' und R' die oben angegebene Bedeutung besitzen, gegebenenfalls mit Alkali verseift, durch Zugabe von Mineralsäure die Carboxylverbindung in Freiheit setzt und nach Kristallisation, wenn erforderlich, durch Zugabe von Base die entsprechenden Salze gewinnt;

c) eine Verbindung der allgemeinen Formel (5)

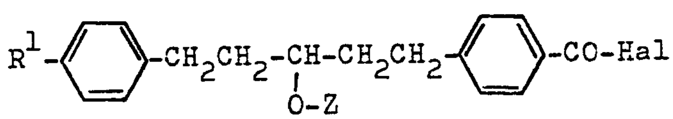

(5)

in der R' die oben angegebene Bedeutung besitzt, Z eine Schutzgruppe, z.B. Acetyl darstellt und Hal ein Halogenatom, vorzugsweise Chlor bedeutet, mit Natriumglycinat in an sich bekannter Weise umsetzt, anschließend mit Alkali die Schutzgruppe abspaltet, durch Zugabe von Mineralsäure die Carboxylverbindung in Freiheit setzt und nach Kristallisation gegebenenfalls mit Base zu den entsprechenden Salzen umsetzt;

d) eine Verbindung der allgemeinen Formel (6)

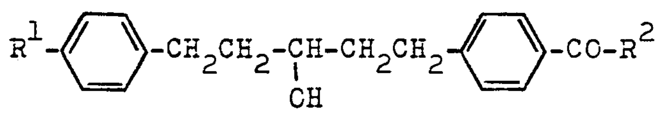

(6)

in der $R^1$ und $R^2$ die oben angegebene Bedeutung besitzen, einer Oxidation mit Dimethylsulfoxid unterzieht, das Oxidationsprodukt kristallisiert und gegebenenfalls die entsprechende Carboxylverbindung mit Base zu den Salzen umsetzt.

2. Arzneimittel, **gekennzeichnet** durch den Gehalt einer nach Anspruch 1 hergestellten Verbindung neben pharmazeutisch anwendbaren Verdünnungs-oder Trägermitteln.

Europäisches Patentamt

# EUROPÄISCHER RECHERCHENBERICHT

Nummer der Anmeldung

EP 87 10 5487

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| E | DE-A-3 546 324 (KLINGE PHARMA) * ganzes Dokument * --- | 1-3 | C 07 C 65/105<br>C 07 C 65/34<br>C 07 C 69/78<br>C 07 C 103/84<br>A 61 K 31/19<br>A 61 K 31/235 |
| A | WO-A-8 501 295 (MERCK) * Anspruch 1 * --- | 1 | |
| A | EP-A-0 006 735 (BEECHAM) * Anspruch 1 * --- | 1 | |
| A | DE-A-3 408 802 (MERCK) * Anspruch 1 * --- | 1 | |
| A | US-A-4 015 010 (HOULIHAN) * Anspruch 1 * --- | 1 | |
| A | DE-A-2 062 611 (AMERICAN CYANAMID) * Anspruch 1 * --- | 1 | |
| A | GB-A-2 002 763 (KLINKE PHARMA) * Anspruch 1 * & DE - A - 2 735 856 (Kat. D) ----- | 1 | RECHERCHIERTE SACHGEBIETE (Int. Cl.4)<br>C 07 C 65/00<br>C 07 C 69/00<br>C 07 C 103/00 |

Der vorliegende Recherchenbericht wurde für alle Patentansprüche erstellt

| Recherchenort | Abschlußdatum der Recherche | Prüfer |
|---|---|---|
| BERLIN | 30-11-1987 | PROBERT C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)